(19) Europäisches Patentamt
European Patent Office
Office européen des brevets

(11) Veröffentlichungsnummer : **0 078 509**
**B1**

(12) **EUROPÄISCHE PATENTSCHRIFT**

(45) Veröffentlichungstag der Patentschrift : **17.07.85**

(21) Anmeldenummer : **82109972.8**

(22) Anmeldetag : **28.10.82**

(51) Int. Cl.[4] : **C 07 D317/72**, C 07 D317/20, C 07 D317/22, C 07 C175/00, C 07 C 43/178, A 01 N 31/06, A 01 N 49/00

# (54) Acetylenverbindungen, Verfahren zu ihrer Herstellung und ihre Verwendung zur Regulierung des Pflanzenwachstums.

(30) Priorität : **04.11.81 DE 3143721**
**04.11.81 DE 3143720**
**04.11.81 DE 3143722**

(43) Veröffentlichungstag der Anmeldung : **11.05.83 Patentblatt 83/19**

(45) Bekanntmachung des Hinweises auf die Patenterteilung : **17.07.85 Patentblatt 85/29**

(84) Benannte Vertragsstaaten :
**AT BE DE FR GB IT NL**

(56) Entgegenhaltungen :
**FR-A- 2 357 541**
**CHEMICAL ABSTRACTS, Band 92, 1980, Seite 577, Nr. 214940e, Columbus, Ohio, USA, V.B. BERZIN et al.: "Abscisic acid and its analogs. VI. Synthesis of methyl-substituted unsaturated hydroxy acids"**

(73) Patentinhaber : **BASF Aktiengesellschaft**
**Carl-Bosch-Strasse 38**
**D-6700 Ludwigshafen (DE)**

(72) Erfinder : **Bliesener, Jens-Uwe, Dr.**
**Silvanerweg 16**
**D-6705 Deidesheim (DE)**
Erfinder : **Baumann, Manfred, Dr.**
**Im Sennteich 26**
**D-6800 Mannheim (DE)**
Erfinder : **Hoffmann, Werner, Dr.**
**Ringstrasse 11 C**
**D-6708 Neuhofen (DE)**
Erfinder : **Sauter, Hubert, Dr.**
**Neckarpromenade**
**D-6800 Mannheim (DE)**
Erfinder : **Jung, Johann, Dr. Dipl-Landwirt**
**Hardenburgstrasse 19**
**D-6703 Limburgerhof (DE)**

**EP 0 078 509 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Acetylenverbindungen, Verfahren zu ihrer Herstellung, diese Verbindungen enthaltende pflanzenwachstumsregulierende Mittel und ihre Verwendung zur Regulierung des Pflanzenwachstums.

Es ist bekannt, daß das in Pflanzen natürlicherweise vorkommende Phytohormon Abszissinsäure (ABA) regulierend in verschiedene physiologische Prozesse der Pflanzen eingreift. (Die Pharmazie *27*, 619 (1972) ; B.V. Milborrow « Abscissic Acid » in « Phytohormones and Related Compounds — A Comprehensive Treatise » Vol. I, S. 295 ff, Editors : Letham Goodwin and Higgins, Elsevier 1978).

So beeinflußt ABA beispielsweise die Samen- und Knospenruhe, die Fruchtreifung und den Abszissionsprozeß von Früchten und Blättern. Von besonderer Bedeutung ist die Abszissinsäure bei der Regulierung des pflanzlichen Wasserhaushaltes. So wird z. B. bei Trockenheit durch verstärkte Biosynthese die endogene Konzentration von ABA in den Blättern erhöht, dies führt dann zu einer Schließung der Spaltöffnung der Blätter (Stomata) und damit zu einer verminderten Wasserabgabe der Pflanzen über die Stomata (verminderte stomatäre Transpiration). Auf diese Weise kann die Pflanze einer unzureichenden Wasserversorgung entgegenwirken. Allerdings reicht die Wirkung der endogenen ABA bei großer Belastung nicht immer aus, um die Pflanze vor Schädigungen durch Hitze oder Trockenheit zu bewahren.

Exogen zugeführte ABA — z. B. durch Besprühen der Pflanzen mit Lösungen von ABA — führt zu einer verstärkten Schließung der Stomata und damit zu einer erheblich verminderten Transpiration. Die behandelten Pflanzen sind dadurch wesentlich resistenter gegen Hitze- und Trockenheitsstreß als unbehandelte.

Eine Behandlung von Kulturpflanzen mit Transpirationsinhibitoren wäre demnach in der landwirtschaftlichen Praxis von sehr großem Nutzen, da sich mit solch einer Behandlung Schädigungen der Kulturpflanzen durch Hitze- und Trockenheitsstreß, die zu internem Wassermangel, zur Welke, zu Ertragseinbußen oder sogar zum völligen Absterben führen können, vermeiden ließen. Derartige Schädigungen stellen in manchen landwirtschaftlichen Anbaugebieten, insbesondere in ariden Gebieten, die regelmäßig durch Hitze und Trockenheit bedroht sind, ein großes Problem dar. Dort besteht ein dringendes Bedürfnis nach Mitteln zur Verminderung der Transpiration von Kulturpflanzen.

Obwohl exogen applizierte ABA aufgrund ihrer biologischen Wirkung als Transpirationsinhibitor für Kulturpflanzen geeignet ist, hat sie bislang keine Verwendung in der landwirtschaftlichen Praxis gefunden. Der Grund hierfür ist darin zu suchen, daß ausreichende Mengen ABA nicht mit einem im Hinblick auf die angestrebte landwirtschaftliche Zielsetzung vertretbaren technischen Aufwand zur Verfügung gestellt werden können. ABA kommt in Pflanzen nur in sehr geringen Mengen vor und ist nur unter ganz erheblichem Aufwand daraus zu isolieren. Andererseits sind die bekannten Totalsynthesen der Abszissinsäure [s. z. B. : J.W. Cornforth et al., J. Chem. Soc. C, *1968*, 1565 ; D.L. Roberts et al., J. Org. Chem. *33*, 3566 (1968) ; T. Oritani et al., Agric. Biol. Chem. (Tokyo) *34*, 108 (1970) ; J.A. Findlay et al., Can. J. Chem. *49*, 2369 (1971) ; H.J. Mayer et al., Helv. Chim. Acta *59*, 1424 (1976) ; F. Kienzle et al., Helv. Chim. Acta *61*, 2616 (1978)] so schwierig und erfordern einen so hohen technischen und ökonomischen Aufwand, daß sie zur Herstellung von Mitteln zur Regulierung des Pflanzenwachstums, insbesondere zur Herstellung von Mitteln zur Regulierung der Transpiration von Kulturpflanzen, nicht in Betracht kommen.

Es sind in der FR-A-2 357 541 gewisse, mit Carotinoiden verwandte Polyenverbindungen vorgeschlagen worden, die sich als Farbstoffe z. B. für Lebensmittel eignen sollen. Vorprodukte für diese Farbstoffe können auch Acetylenverbindungen sein, die mit den nachfolgend beschriebenen Verbindungen eine gewisse Ähnlichkeit aufweisen. Ein weiterer Hinweis, etwa auf die Verwendbarkeit dieser Vorprodukte zu anderen Zwecken als eben denen eines Vorprodukts, ist der FR-A-2 557 541 aber nicht zu entnehmen. Insbesondere berührt die FR-A nicht die Neuheit der nachstehend näher beschriebenen Verbindungen.

Es wurde nun gefunden, daß Acetylenverbindungen der Formel

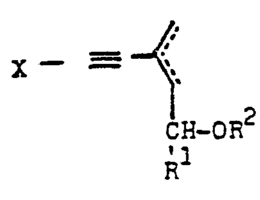

(I)

in der jeweils eine der gestrichelten Linien eine Doppelbindung bedeutet, in der $R^1$ für Wasserstoff oder den Rest $—OR^2$ und $R^2$ für Alkyl mit 1 bis 6 Kohlenstoffatomen stehen oder in der $R^1$ für den Rest $—OR^5$ steht und $R^5$ zusammen mit $R^2$ eine Methylenkette der Formel $—(CH_2)_n—$, die durch eine oder zwei Alkylgruppen mit 1 bis 4 Kohlenstoffatomen substituiert sein kann, bildet, wobei n 2, 3 oder 4 ist, und in der X einen der Reste

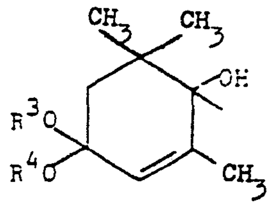 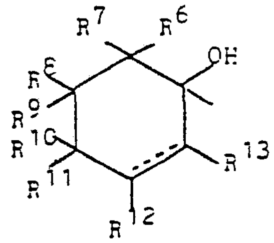 bzw. 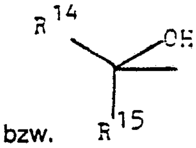

bezeichnet, wobei $R^3$ und $R^4$ gleich sind und jeweils für Alkyl mit 1 bis 6 Kohlenstoffatomen stehen oder zusammen eine Methylenkette der Formel $-(CH_2)_n-$, die durch eine oder zwei Alkylgruppen mit 1 bis 4 Kohlenstoffatomen substituiert sein kann, bilden, wobei n 2, 3 oder 4 ist bzw. $R^6$ für Wasserstoff, Methyl, Ethyl, Isopropyl oder tert.-Butyl, $R^8$ für Wasserstoff, geradkettiges oder gegebenenfalls verzweigtes Alkyl oder Alkenyl mit bis zu 4 Kohlenstoffatomen und $R^7$, $R^9$, $R^{10}$, $R^{11}$, $R^{12}$ und $R^{13}$ jeweils für Wasserstoff oder Methyl stehen, wobei die stereochemische Anordnung der Substituenten $R^6$ bis $R^{13}$ relativ zueinander und relativ zur acetylenischen Seitenkette beliebig ist und die endocyclische gestrichelte Linie eine Doppelbindung bedeuten kann bzw. $R^{14}$ und $R^{15}$ unabhängig voneinander für unverzweigtes oder verzweigtes Alkyl oder Alkenyl mit bis zu 4 Kohlenstoffatomen, Cycloalkyl mit 3 bis 6 Kohlenstoffatomen, unsubstituiertes Phenyl, durch Methoxy, Halogen, Nitro, Cyano oder Alkyl mit 1 bis 4 Kohlenstoffatomen substituiertes Phenyl oder gegebenenfalls durch Methoxy, Halogen, Nitro, Cyano oder Alkyl mit 1 bis 4 Kohlenstoffatomen substituiertes 1-Phenyl-ethyl, 2-Phenylethyl, Benzyl stehen, sehr gute pflanzenwachstumsregulierende, insbesondere transpirationsinhibierende, Wirkung zeigen und in relativ einfacher Weise ohne übermäßig großen technischen Aufwand hergestellt werden können.

Bevorzugte Acetylenverbindungen der Formel I sind solche, bei denen $R^1$ für einen Rest $OR^2$ steht, worin $R^2$ Alkyl mit 1 bis 4 Kohlenstoffatomen bedeutet, weiterhin solche, bei denen $R^1$ für den Rest $-OR^5$ steht und $R^5$ zusammen mit $R^2$ eine Methylenkette der Formel $-(CH_2)_n-$ bildet, die durch Methyl oder Ethyl substituiert sein kann, wobei insbesondere n = 2 bevorzugt ist.

Bevorzugt sind weiterhin Verbindungen, in denen $R^3$ und $R^4$ gleich sind und Alkyl mit 1 bis 4 Kohlenstoffatomen bedeuten, sowie solche, in denen $R^3$ und $R^4$ eine Methylenkette der Formel $-(CH_2)_n-$ bilden, die durch Methyl oder Ethyl substituiert sein kann, wobei insbesondere n = 2 bevorzugt ist.

Bevorzugte Bedeutungen der Substituenten in Formel I sind außerdem :

$R^6 = CH_3$ ; $R^7 = H, CH_3$ ; $R^8 = H, CH_3$ ; $R^9 = H$ ; $R^{10}, R^{11}, R^{12} = H$ ; $R^{13} = CH_3$.

Für $R^{14}$ und $R^{15}$ in Formel I kommen unverzweigte Alkyl- und Alkenylreste mit bis zu 4 Kohlenstoffatomen, wie Methyl, Ethyl, Isopropyl, tert.-Butyl, n-Propyl, Isobutyl, Neopentyl, Allyl, Cycloalkylreste mit 3 bis 6 Kohlenstoffatomen, wie Cyclopentyl, Cyclopropyl, Cyclohexyl, unsubstituiertes Phenyl, durch Methoxy, Halogen, Nitro, Cyano oder Alkyl mit 1 bis 4 Kohlenstoffatomen substituiertes Phenyl, 1-Phenyl-ethyl, 2-Phenyl-ethyl, Benzyl, gegebenenfalls durch Methoxy, Halogen, Nitro, Cyano oder Alkyl mit 1 bis 4 Kohlenstoffatomen substituiert, in Betracht.

Bevorzugt sind Verbindungen der Formel I, bei denen $R^{14}$ Isopropyl, tert.-Butyl oder Cyclopropyl und $R^{15}$ Alkyl mit 1 bis 4 Kohlenstoffatomen, wie Methyl, Ethyl, n-Propyl, Isopropyl, tert.-Butyl, Cyclopropyl oder Benzyl bedeuten.

Man kann die Acetylenverbindungen der Formel I durch Umsetzung eines entsprechenden Ketons, beispielsweise des Ketons II

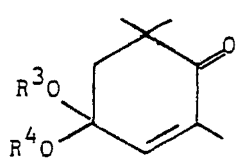 (II)

mit einer Acetylenverbindung III

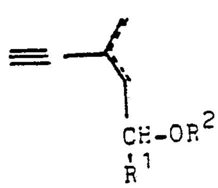 (III)

erhalten ; diese Umsetzung verläuft in Gegenwart eines inerten Lösungs- oder Verdünnungsmittels und in Gegenwart einer Base als Kondensationsmittel.

Als basisches Kondensationsmittel kann Alkalihydroxid, wie KOH, Alkalialkoholat, wie $NaOCH_3$, $KOC_2H_5$, K-tert.-Butylat, Alkaliorganyle, wie n-Butyllithium, Erdalkaliorganyle, wie $CH_3MgCl$, $CH_3MgBr$, Alkalihydrid, wie KH und NaH, sowie Alkaliamid, wie $NaNH_2$ und $KNH_2$, verwendet werden. Vorzugsweise werden als basische Kondensationsmittel $CH_3MgCl$, KOH und Kaliumisobutylat verwendet.

Geeignete inerte Lösungs- oder Verdünnungsmittel sind Ether, wie Diethylether, Diisopropylether, Diethylenglykol-dimethylether, Tetrahydrofuran ; aliphatische Kohlenwasserstoffe, wie Benzol, Toluol, Xylol ; Amide, wie Dimethylformamid, N-Methylpyrrolidon, Hexamethylenphosphorsäuretriamid ; Amine, wie Ammoniak.

Man kann so vorgehen, daß man die Verbindung der Formel III zu der Suspension oder Lösung des Kondensationsmittels hinzufügt, das Keton zugibt und ausreagieren läßt.

Die Reaktion verläuft manchmal schon bei sehr niederer Temperatur ; im allgemeinen liegt sie zwischen − 20 und + 65 °C. Sie kann je nach Reaktionspartner, Kondensationsmittel oder Temperatur, von wenigen Stunden bis zu mehreren Tagen betragen. Die Aufarbeitung des Reaktionsgemisches erfolgt auf übliche Weise, z. B. durch Auswaschen der anorganischen Anteile, gegebenenfalls nach Neutralisation mit Säuren.

Die als Ausgangsverbindungen benötigten Ketone der Formel II sind zum Teil bekannt (US-PS 4 126 641) und können im übrigen z. B. durch Monoketalisierung von 2,6,6-Trimethylcyclohex-2-en-1,4-dion aus bekannten Vorprodukten nach bekannten Methoden hergestellt werden (US-PS 4 076 854 ; Houben-Weyl, Methoden der Organischen Chemie, Band VI/3, S. 199 ff, Georg Thieme-Verlag, Stuttgart 1965).

Die Ausgangsverbindungen der Formel III, bei denen $R^1$ nicht für Wasserstoff steht, sind bekannt (Bull. Chem. Soc. Jap. *50*, 1584 (1977)) und nach bekannten Acetalisierungsmethoden aus 3-Methyl-2-penten-4-inal leicht zugänglich (Bull. Chem. Soc. Jap. *49*, 292 (1976)).

Verbindungen der Formel III, bei denen $R^1$ Wasserstoff bedeutet, sind ebenfalls bekannt bzw. nach bekannten Veretherungsmethoden aus 3-Methyl-2-penten-4-inol zugänglich (J. Organomet. Chem. *117*, 201 (1976).

Darüber hinaus können, sämtliche Verbindungen der Formel III auch durch Dehydratisierung der entsprechenden Carbinole der Formel

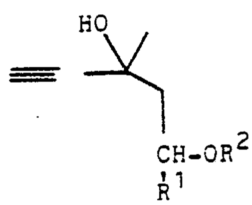

(IV)

in der $R^1$ und $R^2$ die gleichen Bedeutungen wie in Formel III haben, nach an sich bekannten Methoden hergestellt werden.

Die Carbinole der Formel IV lassen sich ihrerseits wiederum leicht in bekannter Weise durch Ethinylierung der entsprechenden Methylketone herstellen (Houben-Weyl, Methoden der Organischen Chemie, Band IV/2, S. 413 ff, Georg Thieme-Verlag, Stuttgart, 1955).

Die Verbindungen der Formel III fallen je nach Herstellungsverfahren in der Regel als Gemisch isomerer Verbindungen an, die in üblicher Weise, z. B. durch Chromatographie, getrennt werden können. Vor Verwendung der Verbindungen der Formel III zur Herstellung der Acetylenverbindungen der Formel I ist eine Trennung der Isomeren im allgemeinen nicht erforderlich.

Die isomeren Komponenten der Formel III werden im folgenden als Gem.-III entsprechend der Formel

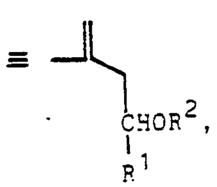

als Z-III entsprechend der Formel

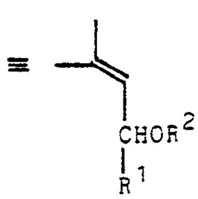

und als E-III entsprechend der Formel

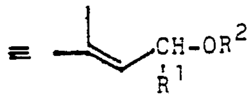

bezeichnet.

Die Acetalisierung von 3-Methyl-2-penten-4-inal und die Veretherung von 3-Methyl-2-penten-4-inol führen im allgemeinen zu Gemischen der Formen Z-III/E-III, ohne daß Gem.-III in nennenswerten Anteilen entsteht. Demgegenüber entstehen bei der Dehydratisierung von Verbindungen der Formel V im allgemeinen zusätzlich größere Anteile geminal substituierter Ethylene der Form Gem.-III.

Je nach Art der Substituenten $R^1$ und $R^2$ können die genannten Isomeren Gem.-III, Z-III und E-III ihrerseits jeweils wieder aus mehreren Isomeren bestehen, z. B. wenn $R^1$ und/oder $R^2$ chirale Kohlenstoffatome enthalten. Die Auftrennung dieser Isomeren ist ebenfalls nach üblichen Methoden möglich, für die Verwendung der Verbindungen zur Herstellung der Acetylenverbindungen der Formel I jedoch in der Regel nicht erforderlich.

Dementsprechend können die Acetylenverbindungen der Formel I ebenfalls als Gemisch mehrerer Isomere auftreten. Hierbei entsprechen im allgemeinen die Isomerenverhältnisse in der Seitenkette denjenigen der als Ausgangsverbindungen jeweils eingesetzten Verbindungen der Formel III, d. h., je nach den relativen Anteilen der zur Herstellung eingesetzten Isomeren Gem.-III, Z-III und E-III erhält man entsprechende Anteile der entsprechenden isomeren Verbindungen der Formeln Gem.-I, Z-I und E-I.

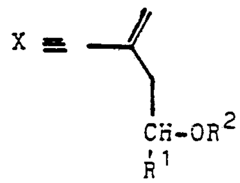

Gem.-I

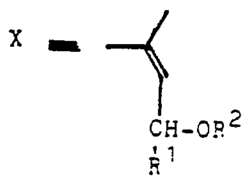

Z-I

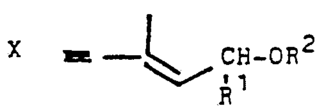

E-I

Je nach Art der Substituenten $R^1$, $R^2$, $R^3$, $R^4$ usw., können die genannten Isomeren Gem.-I, Z-I und E-I ihrerseits jeweils wieder aus mehreren Isomeren bestehen. Ferner gibt die Chiralität des Kohlenstoffatoms, an dem die Hydroxygruppe gebunden ist, Anlaß zum Auftreten von Enantiomeren und gegebenenfalls von Diastereomeren. Alle die genannten Isomeren der Verbindungen der Formel I können durch übliche, geeignete Methoden getrennt werden; für die Anwendung als Mittel zur Regulierung des Pflanzenwachstums werden die Acetylenverbindungen der Formel Z-I bevorzugt; eine Isomerentrennung vor Anwendung ist jedoch üblicherweise nicht erforderlich.

Die folgenden Beispiele erläutern die Herstellung der Acetylenverbindungen der Formel I und der zu ihrer Herstellung nötigen Vorprodukte der Formel III.

Beispiel 1

a) Herstellung von

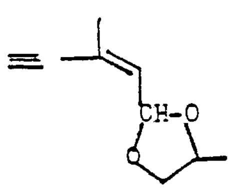

(1a)

Zur siedenden Mischung von 150 g (1,6 Mol) 3-Methyl-2-penten-4-inal und 4,5 g Fumarsäure in 400 ml n-Hexan tropft man unter Rückfluß am Wasserabscheider während zwei Stunden 153 g (2,0 Mol) Propandiol-1,2. Anschließend erhitzt man noch 5 Stunden bis zur Beendigung der Wasserabscheidung, wobei ca. 50 ml Wasser/Propandiol-Gemisch destillativ abgeschieden werden. Am Boden des Reaktionskolbens hat sich nach dem Abkühlen der Reaktionsmischung ein Öl (ca. 150 ml, hauptsächlich Propandiol-1,2) abgesetzt, das verworfen wird. Die überstehende Hexanlösung wird abgetrennt, zweimal mit je 150 ml 5 %iger wäßriger Natriumcarbonatlösung und dann zweimal mit je 150 ml Wasser gewaschen. Nach Trocknung der organischen Phase über Natriumsulfat werden das Hexan unter vermindertem Druck destillativ entfernt und der verbleibende Rückstand (126 g) unter vermindertem Druck destilliert. Nach einem Vorlauf (6 g, Kp 0,4 mbar = 51 bis 56 °C) erhält man 107 g des gewünschten Acetals 1a vom Kp 0,4 mbar = 56 °C. Das Produkt enthält laut $^{1}H$- und $^{13}C$-NMR ca. 85 % der Z-Form und ca. 15 % der E-Form

b) Herstellung von

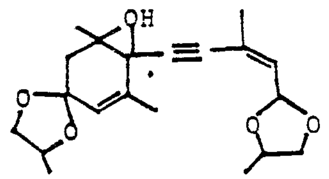

(1)

Zu einer Lösung von 7,5 g Methylmagnesiumchlorid in 67 ml trockenem Tetrahydrofuran tropft man unter Stickstoffatmosphäre bei 0 bis 5 °C unter Rühren 15,2 g des so hergestellten Acetals während 30 Min. hinzu. Danach beläßt man die Reaktionsmischung 3 Stunden bei 20 °C. Dann werden unter Kühlung bei 0 bis 5 °C 17,5 g des Ketons der Formel

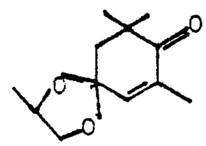

(1b)

während 30 Min. zugetropft und die Mischung anschließend 15 Stunden bei 20 °C gerührt. Man hydrolisiert unter Eiskühlung anschließend durch Zutropfen von 20 ml Wasser, filtriert den Niederschlag ab und befreit das Filtrat unter vermindertem Druck destillativ von Tetrahydrofuran. Den in 200 ml Diethylether aufgenommenen Rückstand wäscht man zweimal mit je 100 ml Wasser, trocknet die etherische Lösung über Natriumsulfat, filtriert und destilliert den Ether unter vermindertem Druck ab. Das verbliebene Öl (28 g) wird sodann einer Kugelrohrdestillation unterzogen, wobei bei 0,005 mbar zwischen 50 und 160 °C unumgesetzte Ausgangsprodukte abdestilliert werden. Zurück bleiben 19,4 g der Verbindung 1. IR (Film) : 2 970, 2 920, 2 865, 1 445, 1 375, 1 345, 1 205, 1 150, 1 085, 1 045, 1 030, 980, 960, $cm^{-1}$.

Laut $^{1}H$-NMR liegen in dem Isomerengemisch wiederum ca. 85 % Z-Isomere und 15 % E-Isomere vor.

Beispiel 2

a) Herstellung von

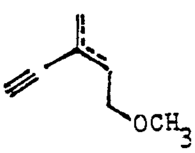

(2a)

Eine Suspension von 50 g wasserfreiem Kupfer (II)-sulfat in 250 ml Paraffinöl wird unter Rühren auf 160 °C erhitzt. Nach Einstellen eines Druckes von 135 mbar werden, während 4 Stunden langsam 300 g 3-Hydroxy-3-methyl-5-methoxy-pentin zur Suspension zugetropft, wobei nach und nach in der Vorlage einer angeschlossenen Destillationsbrücke ein Gemisch aus Wasser, Ausgangsmaterial und Dehydratisierungsprodukt 2a aufgefangen werden. Nach beendeter Zugabe haben sich 180 g Destillat angesammelt, die in 400 ml Diethylether aufgenommen, mit wäßriger Natriumhydrogencarbonatlösung, sodann mit Wasser gewaschen und über Natriumsulfat getrocknet werden. Nach Abdestillieren des Ethers wird der Rückstand unter vermindertem Druck nach Zugabe von 0,5 g Hydrochinon fraktioniert destilliert. Man erhält bei 67 mbar und 52 bis 60 °C 97 g der Verbindung 2a, bestehend aus den Isomeren

der Form Gem.-III, Z-III und E-III im Verhältnis 60 : 30 : 10 entsprechend $^1$H-NMR- und $^{13}$C-NMR-Spektrum.

Als höhersiedende Fraktion werden bei 67 mbar und 61 bis 89 °C nochmals 58 g eines Gemisches aus Ausgangsverbindung und Verbindung 2a aufgefangen, das erneut der Dehydratisierung unterzogen werden kann.

b) Herstellung von

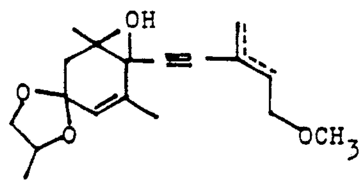

(2)

Entsprechend dem Verfahren nach Beispiel 1b) erhält man aus 7,5 g Methylmagnesiumchlorid, 11 g der Verbindung 2a und 17,5 g 1b nach der Destillation flüchtigerer Bestandteile bei 0,001 mbar und 50 bis 180 °C als Rückstand ein Öl, das laut $^1$H-NMR-Spektrum aus verschiedenen Isomeren 2 besteht (60 % Gem.-2, 30 % Z-2, 10 % E-2). IR (Film) : 2 970, 2 920, 2 870, 1 445, 1 380, 1 370, 1 345, 1 205, 1 090, 1 015, 980, 960, 940 cm$^{-1}$.

## Beispiel 3

Herstellung von

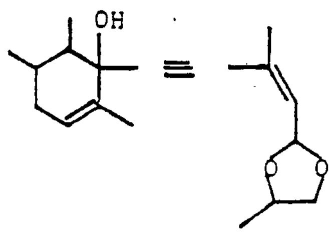

(3)

Zu einer Lösung von 9 g Methylmagnesiumchlorid in 80 ml trockenem Tetrahydrofuran tropft man unter Stickstoffatmosphäre bei 0 bis 5 °C unter Rühren 18,3 g des Acetals 1a während 30 Minuten zu. Danach beläßt man die Reaktionsmischung 3 Stunden bei 20 °C. Dann werden unter Kühlung bei 0 bis 5 °C 13,8 g 2,5,6-Trimethyl-cyclohex-2-en-1-on während 30 Minuten zugetropft ; die Mischung wird anschließend 15 Stunden bei 20 °C gerührt. Man hydrolysiert unter Eiskühlung anschließend durch Zutropfen von 12 ml Wasser, filtriert den Niederschlag ab und befreit das Filtrat unter vermindertem Druck destillativ von Tetrahydrofuran. Den in 200 ml Diethylether aufgenommenen Rückstand wäscht man zweimal mit je 100 ml Wasser, trocknet die etherische Lösung über Natriumsulfat, filtriert und destilliert den Ether unter vermindertem Druck ab. Das verbliebene Öl (28 g) wird sodann einer Kugelrohrdestillation unterzogen, wobei bei 0,005 mbar zwischen 50 und 160 °C unumgesetzte Ausgangsprodukte abdestilliert werden. Bei 0,005 mbar und 205 bis 210 °C destillieren dann 16,0 g der Verbindung 3 über.

IR (Film) : 2 965, 2 925, 2 915, 2 875, 1 445, 1 375, 1 150, 1 080, 1 055, 1 045, 1 025, 960, cm$^{-1}$.

Gemäß $^1$H-NMR-Spektren liegen in dem Isomerengemisch wiederum ca. 85 % Z-Isomere und 15 % E-Isomere vor.

## Beispiel 4

Herstellung von

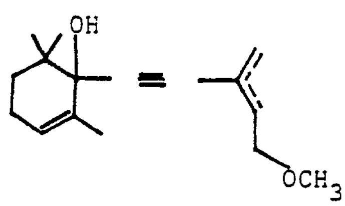

(4)

Entsprechend dem Verfahren nach Beispiel 3b) erhält man aus 13,5 g Methylmagnesiumchlorid, 19,8 g der Verbindung 2a und 20,7 g 2,6,6-Trimethyl-cyclohex-2-en-1-on nach der Destillation flüchtigerer Bestandteile bei 0,001 mbar und 50 bis 120 °C als zweite Fraktion bei gleichem Druck und 125 bis 150 °C ein Öl (25 g), das laut $^1$H-NMR-Spektrum aus verschiedenen Isomeren der Verbindung 4 besteht (60 %

Gem.-4, 30 % Z-4, 10 % E-4). IR (Film) : 2 960, 2 940, 2 915, 2 870, 1 445, 1 375, 1 360, 1 115, 1 090, 1 075, 1 055, 1 030, 1 000, 975, 965 $cm^{-1}$.

Beispiel 5

Herstellung von

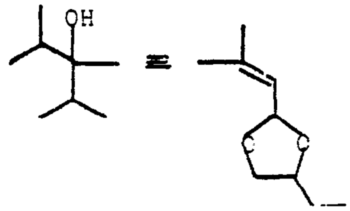

(5)

Zur Lösung von 4,5 g Methylmagnesiumchlorid in 40 ml trockenem Tetrahydrofuran tropft man unter Stickstoffatmosphäre bei 0 bis 5 °C unter Rühren 9,1 g des Acetals 1a während 30 Min. hinzu. Danach beläßt man die Reaktionsmischung 3 Stunden bei 20 °C. Sodann werden unter Kühlung bei 0 bis 5 °C 5,7 g Diisopropylketon während 30 Minuten zugetropft und die Mischung anschließend 15 Stunden bei 20 °C gerührt. Man hydrolysiert unter Eiskühlung anschließend durch Zutropfen von 6 ml Wasser filtriert den Niederschlag ab und befreit das Filtrat unter vermindertem Druck destillativ von Tetrahydrofuran. Den in 200 ml Diethylether aufgenommenen Rückstand wäscht man zweimal mit je 100 ml Wasser, trocknet die etherische Lösung über Natriumsulfat, filtriert und destilliert den Ether unter vermindertem Druck ab. Das verbleibende Öl (13 g) wird sodann einer Kugelrohrdestillation unterzogen, wobei bei 0,005 mbar zwischen 50 und 110 °C unumgesetzte Ausgangsprodukte abdestilliert werden. Zurück bleiben 10,8 g der Verbindung 1b. IR (Film) : 3 470, 2 970, 2 935, 2 875, 1 640, 1 445, 1 380, 1 320, 1 150, 1 055, 1 005, 980, 965, 955, 935 $cm^{-1}$.

Laut $^1H$-NMR liegen in dem Isomerengemisch wiederum ca. 85 % Z-Isomere und 15 % E-Isomere vor.

Beispiel 6

Herstellung von

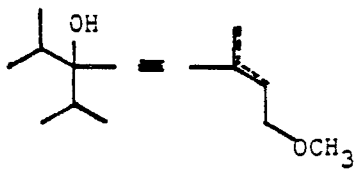

(6)

Entsprechend dem Verfahren nach Beispiel 5b) erhält man aus 5,0 g Methylmagnesiumchlorid, 7,4 g der Verbindung 2a und 64 g Diisopropylketon nach der Destillation flüchtigerer Bestandteile bei 0,001 mbar und 50 bis 80 °C als Rückstand ein Öl (4,0 g), das laut $^1H$-NMR-Spektrum aus verschiedenen Isomeren 6 besteht (60 % Gem.-6, 30 % Z-6, 10 % E-6). IR (Film) : 3 450, 2 970, 1 470, 1 380, 1 150, 1 120, 1 105, 095, 955, 905 $cm^{-1}$.

Als weitere Beispiele für Acetylenverbindungen der Formel I seien im einzelnen die folgenden genannt :

| Verb. Nr. | $R^1$ | $R^2$ | $R^3$ | $R^4$ | IR (Film) $cm^{-1}$ | Bemerkung (Anteil Z:E) |
|---|---|---|---|---|---|---|
| 7 | $O-CH_2-C(CH_3)_2-CH_2-$ | | $-CH_2-C(CH_3)_2-CH_2-$ | | 2945, 2870, 1630, 1465, 1390, 1360, 1195, 1095, 1020, 1010, 980, 960, 925 | 90 % Z 10 % E |
| 8 | $-O-CH(CH_3)-CH_2-$ | | n-Butyl | n-Butyl | | |
| 9 | $-O-CH(CH_3)-CH_2-$ | | n-Hexyl | n-Hexyl | | |
| 10 | n-Butyl | n-Butyl | $-CH_2-CH(CH_3)-$ | | | |
| 11 | n-Hexyl | n-Hexyl | $-CH_2-CH(CH_3)-$ | | | |
| 12 | n-Hexyl | H | $-CH_2-CH(CH_3)-$ | | | |

| Beispiel Nr. | $R^2$ | $R^1$ | $R^6$ | $R^7$ | $R^8$ | $R^9$ | $R^{10}$ | $R^{11}$ | $R^{12}$ | $R^{13}$ | endocycl. Doppelbindung | Isomerenverhältnis Gem/Z/E |
|---|---|---|---|---|---|---|---|---|---|---|---|---|
| 13 | $CH_3$ | $-OCH_3$ | $CH_3$ | $CH_3$ | H | H | H | H | H | $CH_3$ | ja | 0/90/10 |
| IR (Film): 2965, 2920, 2825, 1440, 1375, 1360, 1185, 1130, 1065, 1050, 1000, 950 $cm^{-1}$ | | | | | | | | | | | | |
| 14 | $-CH_2-C(CH_3)_2-CH_2-O-$ | | $CH_3$ | $CH_3$ | H | H | H | H | H | $CH_3$ | nein | 0/90/10 |
| IR (Film): 2950, 2930, 2860, 2845, 1455, 1390, 1195, 1090, 1025, 1010, 980, 955, 925 $cm^{-1}$ | | | | | | | | | | | | |
| 15 | $-CH_2-CHCH_3-O-$ | | $CH_3$ | H | H | H | H | H | H | $CH_3$ | nein | 0/85/15 |
| IR (Film): 2955, 2920, 2865, 2850, 1455, 1440, 1370, 1310, 1145, 1035, 955, 935 $cm^{-1}$ | | | | | | | | | | | | |
| 16 | $-CH_2-CHCH_3-O-$ | | $CH_3$ | H | $CH_3$ | H | H | H | H | H | nein | 0/85/15 |
| IR (Film): 2965, 2920, 2870, 1450, 1375, 1145, 1055, 1050, 1030, 1005, 990, 960, 935 $cm^{-1}$ | | | | | | | | | | | | |
| 17 | $-CH_2-CH_2-O-$ | | $CH_3$ | $CH_3$ | H | H | H | H | H | $CH_3$ | nein | 0/80/20 |
| IR (Film): 2960, 2930, 2880, 1455, 1445, 1375, 1355, 1335, 1325, 1145, 1125, 1060, 1030, 950 $cm^{-1}$ | | | | | | | | | | | | |
| 18 | $-CH_2-CHCH_3-O-$ | | H | H | $CH_3$ | $CH_3$ | H | H | $CH_3$ | H | ja | 0/85/15 |
| IR (Film): 2945, 2920, 2885, 2860, 1445, 1370, 1360, 1145, 1070, 1040, 1010, 990, 960, 930 $cm^{-1}$ | | | | | | | | | | | | |
| 19 | $-CH_2-CHCH_3-O-$ | | H | H | $-C(=CH_2)CH_3$ | H | H | H | H | $CH_3$ | ja | 0/85/15 |
| IR (Film): 2970, 2920, 2880, 1450, 1375, 1150, 1035, 955, 890 $cm^{-1}$ | | | | | | | | | | | | |
| 20 | $-CH_2-CHCH_3-O-$ | | H | H | H | H | H | H | H | H | ja | 0/85/15 |
| IR (Film): 2965, 2920, 1435, 1370, 1310, 1195, 1140, 1040, 950, 925 $cm^{-1}$ | | | | | | | | | | | | |
| 21 | $-CH_2-CHCH_3-O-$ | | H | H | H | H | $CH_3$ | $CH_3$ | H | $CH_3$ | ja | 0/85/15 |
| IR (Film): 2950, 2860, 1440, 1370, 1350, 1305, 1140, 1040, 1015, 1000, 955, 925 $cm^{-1}$ | | | | | | | | | | | | |
| 22 | $-CH_2CHCH_3-O-$ | | $-CH(CH_3)_2$ | H | H | H | H | H | H | H | nein | 0/85/15 |
| IR (Film): 2930, 2865, 1435, 1370, 1355, 1310, 1140, 1115, 1085, 1040, 995, 960, 930 $cm^{-1}$ | | | | | | | | | | | | |

| Beispiel Nr. | $R^2$ | $R^1$ | $R^6$ | $R^7$ | $R^8$ | $R^9$ | $R^{10}$ | $R^{11}$ | $R^{12}$ | $R^{13}$ | endocycl. Doppelbindung | Isomerenverhältnis Gem/Z/E |
|---|---|---|---|---|---|---|---|---|---|---|---|---|
| 23 | $-CH_2-CHCH_3-O-$ | | $-C(CH_3)_2$ | H | H | H | H | H | H | H | nein | 0/85/15 |
| IR (Film): 2930, 2865, 1435, 1385, 1370, 1355, 1135, 1075, 1040, 960, 935 $cm^{-1}$ | | | | | | | | | | | | |
| 24 | $-CH_2CHCH_3-O-$ | | H | H | H | H | H | H | H | H | nein | 0/85/15 |
| IR (Film): 2965, 2930, 2850, 1435, 1365, 1330, 1320, 1305, 1140, 1120, 1045, 990, 955, 930 $cm^{-1}$ | | | | | | | | | | | | |
| 25 | $-CH_2-CHCH_3-O-$ | | H | $CH_3$ | H | H | H | H | H | $CH_3$ | ja | 0/85/15 |
| IR (Film): 2980, 2935, 2890, 1455, 1385, 1330, 1155, 1050, 1010, 970 $cm^{-1}$ | | | | | | | | | | | | |
| 26 | $CH_3$ | H | H | H | H | H | H | H | H | H | ja | 60/30/10 |
| IR (Film): 3414, 2932, 2866, 2838, 1376, 1358, 1108, 1060, 960 $cm^{-1}$ | | | | | | | | | | | | |
| 27 | $CH_3$ | H | $CH_3$ | $CH_3$ | $CH_3$ | H | H | H | H | $CH_3$ | ja | 60/30/10 |
| IR (Film): 3455, 2974, 2957, 2881, 1453, 1387, 1376, 1102, 1061, 1014 $cm^{1}$ | | | | | | | | | | | | |
| 28 | $CH_3$ | H | $CH_3$ | H | H | H | H | H | H | $CH_3$ | ja | 60/30/10 |
| IR (Film): 3433, 2973, 2929, 2875, 1452, 1376, 1110, 1076, 1054, 1004 $cm^{-1}$ | | | | | | | | | | | | |
| 29 | $CH_3$ | H | $CH_3$ | $CH_3$ | H | H | H | H | H | $CH_3$ | ja | 60/30/10 |
| IR (Film): 2960, 2920, 1440, 1370, 1350, 1110, 1070, 1050, 1035, 995, 975, 960 $cm^{-1}$ | | | | | | | | | | | | |
| 30 | $CH_3$ | H | $CH_3$ | H | $CH_3$ | H | H | H | H | $CH_3$ | ja | 60/30/10 |
| IR (Film: 2980, 2960, 2930, 2890, 2835, 1455, 1385, 1120, 1015, 1005, 915, 905 $cm^{-1}$ | | | | | | | | | | | | |
| 31 | $CH_3$ | H | $CH_3$ | $CH_3$ | H | H | H | H | H | $CH_3$ | nein | 60/30/10 |
| IR (Film): 2950, 2920, 2860, 1450, 1370, 1110, 1070, 1050, 1025, 965, 945 $cm^{-1}$ | | | | | | | | | | | | |
| 32 | $n-C_6H_{13}$ | H | $CH_3$ | $CH_3$ | H | H | H | H | H | $CH_3$ | ja | |

| Beispiel Nr. | $R^2$ | $R^1$ | $R^6$ | $R^7$ | $R^8$ | $R^9$ | $R^{10}$ | $R^{11}$ | $R^{12}$ | $R^{13}$ | endocycl. Doppelbindung | Isomerenverhältnis Gem/Z/E |
|---|---|---|---|---|---|---|---|---|---|---|---|---|
| 33 | n-$C_4H_9$ | H | $CH_3$ | $CH_3$ | H | H | H | H | H | $CH_3$ | Ja | |
| 34 | n-$C_4H_9$ | n-$C_4H_9O$ | $CH_3$ | $CH_3$ | H | H | H | H | H | $CH_3$ | Ja | |
| 35 | n-$C_6H_{13}$ | n-$C_6H_{13}O$ | $CH_3$ | $CH_3$ | H | H | H | H | H | $CH_3$ | Ja | |

| Beispiel Nr. | $R^2$ $R^1$ | $R^{14}$ | $R^{15}$ | IR (Film) [$cm^{-1}$] | Isomerenverhältnis Gem/Z/E |
|---|---|---|---|---|---|
| 36 | $-CH_2-C(CH_3)_2-CH_2-O$ | Isopropyl | Isopropyl | 2955, 2865, 2840, 1465, 1390, 1140, 1090, 1010, 980, 960, 950, 925 | 0/90/10 |
| 37 | $-CH_2-C(CH_3)_2-CH_2-O-$ | Cyclopropyl | Cyclopropyl | 3000, 2950, 2845, 1465, 1445, 1390, 1360, 1305, 1230, 1140, 1085, 1020, 975, 960, 925 | 0/90/10 |
| 38 | $-CH_2-CH(CH_3)-O-$ | Phenyl | Cyclopropyl | 3440, 2980, 2960, 2930, 2885, 1445, 1375, 1150, 1085, 1040, 1005, 965, 925, 755, 705 | 0/85/15 |
| 39 | $-CH_2-CH(CH_3)-O-$ | Cyclopropyl | Cyclopropyl | 3450, 3005, 2975, 2930, 2875, 1440, 1375, 1315, 1145, 1050, 1030, 995, 960, 930 | 0/85/15 |
| 40 | $-CH_2-CH(CH_3)-O-$ | Isopropyl | Phenyl | 2965, 2920, 2870, 1440, 1370, 1145, 1055, 1030, 1000, 955, 935, 755, 700 | 0/85/15 |
| 41 | $-CH_2-CH(CH_3)-O-$ | Cyclopropyl | 4-Methoxyphenyl | 2975, 1605, 1505, 1375, 1295, 1245, 1170, 1145, 1030, 990, 965, 830 | 0/85/15 |
| 42 | $-CH_2-CH(CH_3)-O-$ | Cyclopentyl | Phenyl | 2950, 2865, 1440, 1370, 1145, 1030, 995, 960, 930, 700 | 0/85/15 |
| 43 | $-CH_2-CH(CH_3)-O-$ | t-Butyl | Methyl | 3450, 2960, 2920, 2865, 1445, 1370, 1315, 1190, 1140, 1080, 1050, 1005, 960, 935, 905 | 0/85/15 |
| 44 | $-CH_2-CH(CH_3)-O-$ | Ethyl | t-Butyl | 3460, 2960, 2920, 2865, 1440, 1385, 1370, 1355, 1135, 1100, 1045, 990, 955 | 0/85/15 |
| 45 | $-CH_2-CH(CH_3)-O-$ | Isobutyl | Isobutyl | 3450, 2945, 2920, 2860, 1460, 1440, 1375, 1360, 1145, 1050, 1005, 960, 930 | 0/85/15 |
| 46 | $-CH_2-CH(CH_3)-O-$ | Methyl | Neopentyl | 3450, 2970, 2945, 2895, 2865, 1460, 1445, 1390, 1375, 1360, 1145, 1105, 1075, 1050, 1015, 995, 960, 930 | 0/85/15 |

| Beispiel Nr. | $R^2$ | $R^1$ | $R^{14}$ | $R^{15}$ | IR (Film) [$cm^{-1}$] | Isomerenverhältnis Gem/Z/E |
|---|---|---|---|---|---|---|
| 47 | $-CH_2-CH(CH_3)-O-$ | | Propen-2-yl | Propen-2-yl | 2975, 2925, 2875, 1635, 1440, 1370, 1145, 1125, 1050, 990, 960, 935, 905 | 0/85/15 |
| 48 | $-CH_2-CH(CH_3)-O-$ | | 1-Propyl | Ethyl | 3450, 2970, 2940, 2885, 1450, 1375, 1325, 1145, 1055, 1000, 960 | 0/85/15 |
| 49 | $-CH_2-CH(CH_3)-O-$ | | Isobutyl | Methyl | 3450, 2970, 2950, 2920, 2865, 1440, 1375, 1360, 1145, 1080, 1050, 995, 960, 930 | 0/85/15 |
| 50 | $-CH_2-CH(CH_3)-O-$ | | 1-Propyl | 1-Propyl | 3450, 2955, 2925, 2865, 1440, 1370, 1135, 1070, 1050, 990, 975, 955, 930 | 0/85/15 |
| 51 | $-CH_2-CH(CH_3)-O-$ | | 2-Phenyl--ethyl (2)- | Isopropyl | 2960, 2920, 2870, 1445, 1375, 1140, 1050, 1020, 995, 980, 960, 760, 700 | 0/85/15 |

Die Acetylenverbindungen der Formel I greifen in den Metabolismus der Pflanzen ein und können deshalb als Wachstumsregulatoren eingesetzt werden.

Für die Wirkungsweise von Pflanzenwachstumsregulatoren gilt nach den bisherigen Erfahrungen, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z. B. Sonnenscheindauer, Durchschnittstemperatur, Niederschlagsmenge,
e) von der Bodenbeschaffenheit (einschließlich Düngung),
f) von der Formulierung bzw. Anwendungsform des Wirkstoffs und schließlich,
g) von den angewendeten Konzentrationen der aktiven Substanz.

In jedem Falle sollen Wachstumsregulatoren die Kulturpflanzen in gewünschter Weise positiv beeinflussen.

Aus der Reihe der verschiedenartigen Anwendungsmöglichkeiten der erfindungsgemäßen Pflanzenwachstumsregulatoren im Pflanzenbau, in der Landwirtschaft und im Gartenbau, werden einige beispielhaft erläutert :

A. Mit den erfindungsgemäßen Verbindungen läßt sich die Transpiration von Kulturpflanzen beeinflussen. Die Behandlung mit diesen Verbindungen führt zu einer verstärkten Schließung der Stomata und damit zu einer erheblich verminderten Transpiration. Die behandelten Pflanzen sind dadurch wesentlich resistenter gegen Trockenheitsstreß als unbehandelte. Damit lassen sich Schädigungen der Kulturpflanzen durch diesen Streßfaktor, der zu Ertragseinbußen oder sogar zum völligen Absterben führen kann, vermeiden und eine Regulierung des Wasserhaushaltes erreichen.

B. Mit den erfindungsgemäßen Verbindungen läßt sich auch das vegetative Wachstum der Pflanzen stark hemmen, was sich insbesondere in einer Reduzierung des Längenwachstums äußert. Die behandelten Pflanzen weisen demgemäß einen gedrungenen Wuchs aus ; außerdem ist eine dunklere Blattfärbung zu beobachten.

Als vorteilhaft für die Praxis erweist sich z. B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des « Lagerns » (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.

Ein weiterer Mechanismus der Ertragssteigerung mit Wachstumshemmern beruht darauf, daß die Nährstoffe in stärkerem Maße der Blüten- und Fruchtbildung zugute kommen, während das vegetative Wachstum eingeschränkt wird. Ferner kann so wegen der relativ geringen Blatt- bzw. Pflanzenmasse dem Befall mit verschiedenen, insbesondere pilzlichen Krankheiten vorgebeugt werden.

Die Hemmung des vegetativen Wachstums ermöglicht außerdem bei vielen Kulturpflanzen eine dichtere Bepflanzung, so daß ein Mehrertrag, bezogen auf die Bodenfläche, erzielt werden kann. Die erfindungsgemäßen Verbindungen eignen sich besonders zur Hemmung des vegetativen Wachstums bei Kulturpflanzen, wie Soja, Sonnenblumen, Erdnüssen, Raps, Zierpflanzen, Baumwolle, Reis, Weizen, Gerste und Gräsern.

C. Mit den neuen Wirkstoffen lassen sich Mehrerträge sowohl an Pflanzenteilen als auch an pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die neuen Stoffe Ertragssteigerungen durch Eingriffe in den Pflanzlichen Stoffwechsel bzw. durch Förderung oder Hemmung des vegetativen und/oder des generativen Wachstums verursachen.

D. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen. Mit den erfindungsgemäßen Verbindungen läßt sich insbesondere eine Beschleunigung der Seneszenz erzielen.

Von wirtschaftlicher Bedeutung ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schlenobst ermöglicht wird. Der gleiche Mechanismus, d. h. die Förderung der Ausbildung von Trenngewebe zwischen Frucht- bzw. Blatt und Sproßteil der Pflanze, ist auch für ein gut kontrollierbares Entblättern der Bäume wesentlich. Diese Wirkungsart ist bei den erfindungsgemäßen Verbindungen besonders ausgeprägt.

Die Wirkung der Acetylenverbindungen zeigt sich sowohl bei Monokotylen, z. B. bei Getreide, wie Weizen, Gerste, Roggen, Hafer, Sorghum und Reis oder Mais oder Gräsern als auch Dikotylen, z. B. Sonnenblumen, Tomaten, Erdnüssen, Reben, Baumwolle, Raps, Zuckerrüben und Soja, und verschiedenen Zierpflanzen, wie Chrysanthemen, Poinsettien und Hibiskus.

Die erfindungsgemäßen Wirkstoffe können den Kulturpflanzen sowohl vom Samen her (als Saatgutbeizmittel) als auch über den Boden, d. h. durch die Wurzel, sowie insbesondere durch Spritzung über das Blatt zugeführt werden. Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Aufwandmengen von 0,001 bis 12 kg Wirkstoff, vorzugsweise 0,01 bis 3 kg/ha, ausreichend.

Die erfindungsgemäßen Mittel können in Form üblicher Formulierungen angewendet werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als

Hilfsstoffe zur Formulierung kommen im wesentlichen Lösungsmittel, wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Ketone (z. B. Cyclohexanon), Dimethylformamid und Wasser in Betracht ; feste Trägerstoffe wie natürliche Gesteinsmehle (z. B. Kaoline, Tonerden, Talkum, Kreide) und synthetische Gesteinsmehle (z. B. hochdisperse Kieselsäure, Silikate) ; Emulgiermittel oder sonstige oberflächenaktive Mittel, wie nichtionogene und anionische Emulgatoren (z. B. Polyoxyethylen-Fettalkohol-Ether, Alkylsulfonate) und Dispergiermittel wie Lignin, Sulfitablaugen und Methylcellulose.

Die Formulierungen enthalten im allgemeinen zwischen 0,1 und 95 Gew.-% Wirkstoff, vorzugsweise zwischen 0,5 und 90 Gew.-%.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind :

I. 20 Gewichtsteile der Verbindung des Beispiels 1 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

II. 3 Gewichtsteile der Verbindung des Beispiels 1 werden mit 97 Gewichtsteilen feinteiligem Kaolin innig vermischt. Man erhält auf diese Weise ein Stäubemittel, das 3 Gew.-% des Wirkstoffs enthält.

III. 30 Gewichtsteile der Verbindung des Beispiels 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gewichtsteile der Verbindung des Beispiels 3 werden mit 10 Teilen Natriumsalze eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

V. 20 Teile der Verbindung des Beispiels 3 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung des Beispiels 1 mit 10 Gewichtsteilen N-Methylpyrrolidon und erhält eine Lösung, die zur Anwendung in Form kleinster Tropfen geeignet ist.

VII. 20 Gewichtsteile der Verbindung des Beispiels 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

VIII. 20 Gewichtsteile des Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d. h. die Wirksamkeit des Kombinationsproduktes ist größer als die addierten Wirksamkeiten der Einzelkomponenten.

Fungizide, die mit den erfindungsgemäßen Verbindungen kombiniert werden können, sind beispielsweise Dithiocarbamate und deren Derivate, wie

Ferridimethyldithiocarbamat,

Zinkdimethyldithiocarbamat,

Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamoyl)-disulfid ;

Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Strukturen, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
O,O-Diethyl-phthalimidophosphonthionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2)-benzimidazol,
Piperazin-1,4-diyl-bis-1-(2,2,2-trichlor-ethyl)-formamid,
2-Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)-alaninat,
D,L-N-(2,6-Dimethyl-phenyl)-N-(2'-methoxyacetyl)-alaninmethylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

In den folgenden Beispielen wird die Wirkung der erfindungsgemäß verwendbaren Acetylenverbindungen der Formel I als Pflanzenwachstumsregulatoren beispielhaft gezeigt, ohne die Möglichkeit weiterer Anwendungen als Wachstumsregulatoren auszuschließen.

Die transpirationsinhibierende Wirkung der Acetylenverbindungen der Formel I kann beispielsweise.

durch Welkebonitur bei Trockenstreß,
durch Bestimmung des Wasserverbrauchs und
durch Messung des Diffusionswiderstandes

gezeigt werden.

1. Welkeverhalten bei Trockenstreß (Gewächshausversuch)

Pflanzen, z. B. Gerste, werden im üblichen Verfahren auf ausreichend mit Nährstoffen versorgtem Torfkultursubstrat in Kunststoffgefäßen von ca. 12,5 cm Durchmesser bis zur Blattspritzung bei voller Wassersättigung des Substrates angezogen. Die Aufwandmengen betragen 0,2 bzw. 0,1 mg Wirkstoff pro Gefäß. Nach der Applikation der wäßrig aufbereiteten Substanzen werden die Gefäße in trockene Paletten ohne weitere Wasserzufuhr gesetzt. Die auftretende Welkung der Pflanzen wird bonitiert (Bonitierungsnote O bedeutet keine Welke, 9 bedeutet totale Welke).

In diesem Test zeigen die Wirkstoffe Nr. 1, 2, 3, 4, 5, 7 bis 33, 40, 43, 47, 50, 55, 56, 57, 58 und 59 eine gute transpirationsinhibierende Wirkung.

2. Bestimmung des Wasserverbrauchs (Laborkurzzeitversuch)

Junge Sonnenblumen, die entsprechend dem obigen Versuch angezogen wurden, werden bei einer Wuchshöhe von ca. 25 cm mit wäßrigen Aufbereitungen der Prüfsubstanzen besprüht, unmittelbar danach ca. 10 cm unterhalb des Vegetationszentrums abgeschnitten und in mit Wasser gefüllte, graduierte (Zentrifugen-)-Röhrchen gestellt. Die Aufwandmengen betragen 0,2 bzw. 0,1 mg Wirkstoff pro Gefäß. Bei Raumtemperatur, diffusem Licht und zugluftfrei wird in Zeitintervallen die Wasserabnahme durch Ablesung an der Graduierung festgestellt. Bei Versuchsende (nach 24 Stunden) wird die Blattfläche der Versuchsglieder mit dem Blattflächenmeßgerät der Fa. LJ-COR Inc. gemessen, der Wasserverbrauch wird in $\mu l/cm^2$ ausgewiesen.

In diesem Test zeigt sich, daß der Wasserverbrauch der Pflanzen, die mit den Wirkstoffen Nr. 1, 2, 3 und 7 behandelt wurden, wesentlich niedriger ist als der unbehandelter Pflanzen.

3. Messung des Diffusionswiderstandes (Gewächshausversuch)

Testpflanzen (Sonnenblumen, Soja) werden wie oben beschrieben angezogen und mit wäßrigen Aufbereitungen der Substanzen besprüht und bei normaler Wasserzufuhr im Gewächshaus weitergezogen. Die Aufwandmengen betragen 0,2 bzw. 0,1 mg Wirkstoff pro Gefäß. Der Diffusionswiderstand der Blätter — als Parameter für den Öffnungszustand der Stomata — wird mittels Autoporometer bestimmt.

In diesem Test zeigt sich, daß der Diffusionswiderstand der Blätter der Pflanzen, die mit den Wirkstoffen Nr. 1, 2, 3 und 7 behandelt wurden, höher ist als der der Blätter unbehandelter Pflanzen.

4. Wachstumsregulierende Eigenschaften

Zur Bestimmung der wachstumsregulierenden Eigenschaften der Acetylenverbindungen der Formel I werden Testpflanzen auf ausreichend mit Nährstoffen versorgtem Kultursubstrat in Kunststoffgefäßen von ca. 12,5 cm Durchmesser angezogen.

Im Vorauflaufverfahren werden die Testsubstanzen in wäßriger Aufbereitung am Tage der Einsaat auf das Saatbett gegossen.

Im Nachauflaufverfahren werden die zu prüfenden Substanzen in wäßriger Aufbereitung auf die Pflanzen gesprüht. Die beobachtete wachstumsregulierende Wirkung wird bei Versuchsende durch Wuchshöhenmessung belegt. Die so gewonnenen Meßwerte werden zur Wuchshöhe der unbehandelten Pflanzen in Relation gesetzt.

Gleichlaufend zur Reduzierung des Längenwachstums steigt die Farbintensität der Blätter an. Der erhöhte Chlorophyllgehalt läßt eine ebenfalls erhöhte Photosyntheserate und damit eine erhöhte Ertragsbildung erwarten.

In diesem Test zeigen die Wirkstoffe Nr. 1, 2, 3, 4, 15, 17, 19, 24, 25, 27, 29, 30, 31, 32, 33, 47, 48, 50, 51, 52, 53, 54, 56, 57, 59 und 60 sowohl im Nachauflauf- als auch im Vorauflaufverfahren eine ausgeprägte wachstumsregulierende Wirkung.

**Patentansprüche**

1. Acetylenverbindungen der Formel

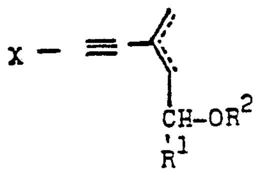

(I)

in der jeweils eine der gestrichelten Linien eine Doppelbindung bedeutet, $R^1$ für Wasserstoff oder den Rest $—OR^2$ und $R^2$ für Alkyl mit 1 bis 6 Kohlenstoffatomen stehen oder in der $R^1$ für den Rest $—OR^5$ steht und $R^5$ zusammen mit $R^2$ eine Methylenkette der Formel $—(CH_2)_n—$, die durch eine oder zwei Alkylgruppen mit 1 bis 4 Kohlenstoffatomen substituiert sein kann, bildet, wobei n 2, 3 oder 4 ist, und in der X einen der Reste

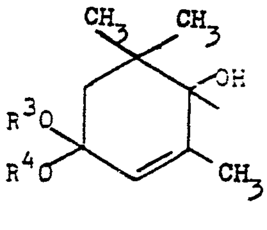

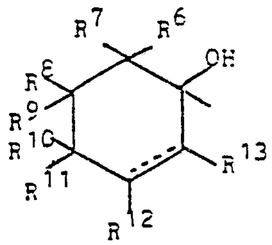

bzw.

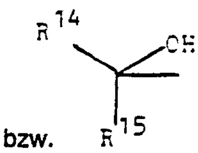

bezeichnet, wobei $R^3$ und $R^4$ gleich sind und jeweils für Alkyl mit 1 bis 6 Kohlenstoffatomen stehen oder zusammen eine Methylenkette der Formel $—(CH_2)_n—$, die durch eine oder zwei Alkylgruppen mit 1 bis 4 Kohlenstoffatomen substituiert sein kann, bilden, wobei n 2, 3 oder 4 ist bzw. $R^6$ für Wasserstoff, Methyl, Ethyl, Isopropyl oder tert.-Butyl, $R^8$ für Wasserstoff, geradkettiges oder gegebenenfalls verzweigtes Alkyl oder Alkenyl mit bis zu 4 Kohlenstoffatomen und $R^7$, $R^9$, $R^{10}$, $R^{11}$, $R^{12}$ und $R^{13}$ jeweils für Wasserstoff oder Methyl stehen, wobei die stereochemische Anordnung der Substituenten $R^6$ bis $R^{13}$ relativ zueinander und relativ zur acetylenischen Seitenkette beliebig ist und die endocyclische gestrichelte Linie eine Doppelbindung bedeuten kann bzw. $R^{14}$ und $R^{15}$ unabhängig voneinander für unverzweigtes Alkyl oder Alkenyl mit bis zu 4 Kohlenstoffatomen, Cycloalkyl mit 3 bis 6 Kohlenstoffatomen, unsubstituiertes Phenyl, durch Methoxy, Halogen, Nitro, Cyano oder Alkyl mit 1 bis 4 Kohlenstoffatomen substituiertes Phenyl oder gegebenenfalls durch Methoxy, Halogen, Nitro, Cyano oder Alkyl mit 1 bis 4 Kohlenstoffatomen substituiertes 1-Phenyl-ethyl, 2-Phenylethyl, Benzyl stehen.

2. Verfahren zur Herstellung von Acetylenverbindungen der Formel I gemäß Anspruch 1, dadurch gekennzeichnet, daß man ein entsprechendes Keton (II)

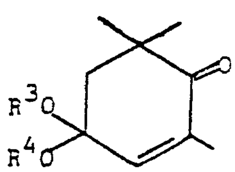

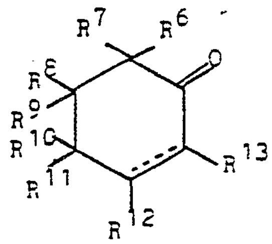

bzw.

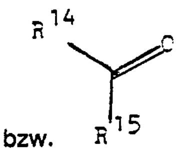

mit einem entsprechenden Acetylenderivat (III)

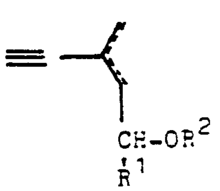

(III)

in Gegenwart eines inerten Lösungs- oder Verdünnungsmittels und einer Base als Kondensationsmittel umsetzt.

3. Mittel zur Regulierung des Pflanzenwachstums, enthaltend inerte Zusatzstoffe und eine Acetylenverbindung der Formel I gemäß Anspruch 1.

4. Mittel zur Verminderung der Transpiration und zur Vermeidung von Beeinträchtigungen der Pflanzen durch Hitze- und Trockenheitsstreß, enthaltend inerte Zusatzstoffe und eine Acetylenverbindung der Formel I gemäß Anspruch 1.

5. Verfahren zur Regulierung des Pflanzenwachstums, dadurch gekennzeichnet, daß man eine wirksame Menge einer Acetylenverbindung der Formel I gemäß Anspruch 1 auf die Pflanzen oder deren Samen einwirken läßt.

6. Verfahren zur Verminderung der Transpiration von Pflanzen, dadurch gekennzeichnet, daß man eine wirksame Menge einer Acetylenverbindung der Formel I gemäß Anspruch 1 auf die Pflanzen oder deren Samen einwirken läßt.

7. Verwendung von Acetylenverbindungen der Formel I gemäß Anspruch 1 zur Verminderung der Transpiration von Pflanzen.

**Claims**

1. An acetylene compound of the formula

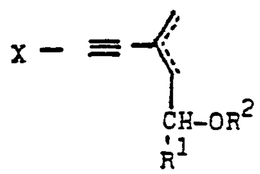

(I)

where one of the broken lines is a double bond, $R^1$ is hydrogen or $—OR^2$ and $R^2$ is alkyl of 1 to 6 carbon atoms, or $R^1$ is $—OR^5$, and $R^5$, together with $R^2$, forms a methylene chain which is of the formula $—(CH_2)_n—$ where n is 2, 3 or 4, and can be monosubstituted or disubstituted by alkyl of 1 to 4 carbon atoms, and X is one of the radicals

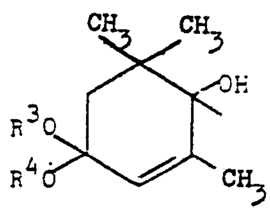

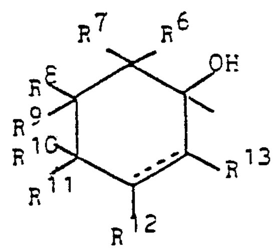

or

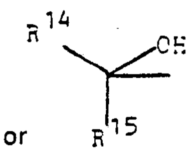

where $R^3$ and $R^4$ are identical and are each alkyl of 1 to 6 carbon atoms, or together form a methylene chain which is of the formula $—(CH_2)_n—$ where n is 2, 3 or 4, and can be monosubstituted or disubstituted by alkyl of 1 to 4 carbon atoms, and $R^6$ is hydrogen, methyl, ethyl, isopropyl or tert.-butyl, $R^8$ is hydrogen, straight-chain or branched alkyl or alkenyl of up to 4 carbon atoms, and $R^7$, $R^9$, $R^{10}$, $R^{11}$, $R^{12}$ and $R^{13}$ are each hydrogen or methyl, substituents $R^6$ to $R^{13}$ being in any stereochemical arrangement relative to each other and relative to the acetylenic side-chain, and the endocyclic broken line optionally denoting a double bond, and $R^{14}$ and $R^{15}$ are, independently of one another, straight-chain alkyl or alkenyl of up to 4 carbon atoms, cycloalkyl of 3 to 6 carbon atoms, unsubstituted phenyl or phenyl substituted by methoxy, halogen, nitro, cyano or alkyl of 1 to 4 carbon atoms, or 1-phenylethyl, 2-phenylethyl or benzyl which is unsubstituted or substituted by methoxy, halogen, nitro, cyano or alkyl of 1 to 4 carbon atoms.

2. A process for manufacturing an acetylene compound of the formula I as claimed in claim 1, wherein an appropriate ketone (II)

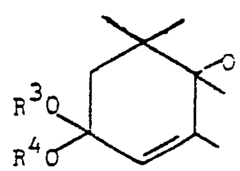

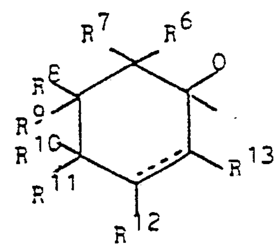

or

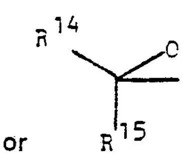

is reacted with an appropriate acetylene derivative (III)

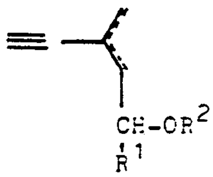

(III)

in the presence of an inert solvent or diluent and in the presence of a base as the condensing agent.

3. An agent for regulating plant growth, containing inert additives and an acetylene compound of the formula I as claimed in claim 1.

4. An agent, containing inert additives and an acetylene compound of the formula I as claimed in claim 1, for reducing transpiration and for preventing damage to plants by heat and water shortage stress.

5. A process for regulating plant growth, wherein an effective amount of an acetylene compound of the formula I as claimed in claim 1 is allowed to act on the plants or their seed.

6. A process for reducing transpiration in plants, wherein an effective amount of an acetylene compound of the formula I as claimed in claim 1 is allowed to act on the plants or their seed.

7. The use of an acetylene compound of the formula I as claimed in claim 1 for reducing transpiration in plants.

**Revendications**

1. Dérivés d'acétylène de formule

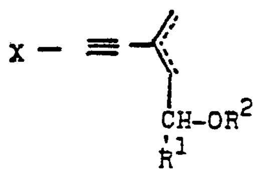

(I)

dans laquelle chacune des lignes à pointillés représente une double liaison, $R^1$ représente hydrogène ou le reste $-OR^2$ et $R^2$ représente alkyle à 1 à 6 atomes de carbone ou bien dans laquelle $R^1$ représente le reste $-OR^5$ et $R^5$ forme avec $R^2$ une chaîne méthylène de formule $-(CH_2)_n$, qui peut être substituée par un ou deux groupes alkyle de 1 à 4 atomes de carbone, n étant 2, 3 ou 4, et dans laquelle X représente un des restes

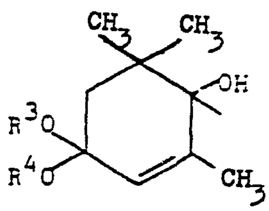

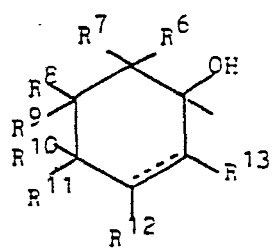

ou

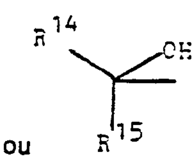

$R^3$ et $R^4$ étant identiques et représentant chacun alkyle de 1 à 6 atomes de carbone ou formant ensemble une chaîne méthylène de formule $-(CH_2)_n-$, qui peut être substituée par un ou deux groupes alkyle à 1 à 4 atomes de carbone, n étant 2, 3 ou 4, et $R^6$ représente hydrogène, méthyle, éthyle, isopropyle ou tert-butyle, $R^8$ représente hydrogène, alkyle ou alcényle ayant jusqu'à 4 atomes de carbone, à chaîne droite ou éventuellement ramifiée, et $R^7$, $R^9$, $R^{10}$, $R^{11}$, $R^{12}$ et $R^{13}$ représentent chacun hydrogène ou méthyle, la disposition stéréochimique des substituants $R^6$ à $R^{13}$ relativement les uns aux autres, ou relativement à la chaîne latérale acétylénique, étant quelconque et la ligne pointillée endocyclique pouvant représenter une double liaison, et $R^{14}$ et $R^{15}$ représentent, indépendamment l'un de l'autre, alkyle ou alcényle non ramifié ayant jusqu'à 4 atomes de carbone, cycloalkyle de 3 à 6 atomes de carbone, phényle non substitué, phényle substitué par méthoxy, halogène, nitro, cyano ou alkyle à 1 à 4 atomes de carbone, ou 1-phényl-éthyle, 2-phényl-éthyle, benzyle, éventuellement substitués par méthoxy, halogène, nitro, cyano ou alkyle à 1 à 4 atomes de carbone.

2. Procédé de préparation des dérivés d'acétylène de formule I selon la revendication 1, caractérisé par le fait qu'on fait agir une cétone correspondante (II)

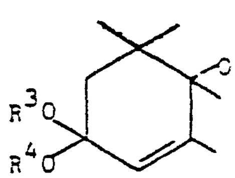

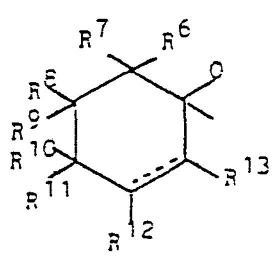

ou

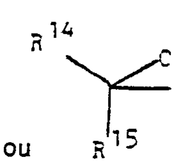

avec un dérivé d'acétylène correspondant (III)

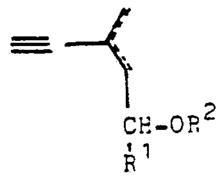

(III)

en présence d'un solvant ou diluant inerte et d'une base comme agent de condensation.

3. Agent de régularisation de la croissance des plantes, contenant des additifs inertes et un dérivé d'acétylène de formule I selon la revendication 1.

4. Agent pour diminuer la transpiration et éviter les préjudices causés aux plantes par les contraintes de chaleur et de sécheresse, contenant des additifs inertes et un dérivé d'acétylène de formule I selon la revendication 1.

5. Procédé de régularisation de la croissance des plantes, caractérisé par le fait que l'on fait agir sur les plantes ou leur semis une quantité efficace d'un dérivé d'acétylène de formule I selon la revendication 1.

6. Procédé pour réduire la transpiration des plantes, caractérisé par le fait que l'on fait agir sur les plantes ou leur semis une quantité efficace d'un dérivé d'acétylène de formule I selon la revendication 1.

7. Utilisation de dérivés d'acétylène de formule I selon la revendication 1 pour diminuer la transpiration des plantes.